# EUROPÄISCHE PATENTANMELDUNG

(11) **EP 2 196 449 A1**
(43) Veröffentlichungstag der Anmeldung: **16.06.2010**
(21) Anmeldenummer: 09178564.2
(22) Anmeldetag: 09.12.2009
(51) Int. Cl.: C07C 29/58, C07C 41/24, C07C 31/38, C07C 43/13

(54) **Verfahren zur Herstellung von Perfluoralkyl- oder Perfluoralkoxyalkanolen**

(30) Priorität: 12.12.2008 DE 102008061722
(71) Anmelder: Saltigo GmbH, 40764 Langenfeld (DE)
(72) Erfinder: Ebenbeck, Wolfgang, 51373 Leverkusen (DE)
(74) Vertreter: Pettrich, Klaus-Günter

(57) **Zusammenfassung**

Die vorliegende Erfindung betrifft ein Verfahren zur Herstellung von Perfluoralkyl- oder Perfluoralkoxyalkanolen.

## Beschreibung

Die vorliegende Erfindung betrifft ein Verfahren zur Herstellung von Perfluoralkyl- oder Perfluoralkoxyalkanolen.

Perfluoralkyl- und Perfluoralkoxyalkanole stellen wertvolle Zwischenprodukte für die Herstellung von Pharmazeutika dar.

Aus der Literatur sind verschiedene Verfahren zur Herstellung von Perfluoralkyl- und Perfluoralkoxyalkanolen bekannt.

Tetrahedron Lett. 35, 9141 (1994) beschreibt ein Verfahren zur Herstellung eines Perfluoralkylalkanols, insbesondere des 4,4,5,5,5-Pentafluor-2-pentan-1-ol, ausgehend von Pentafluorethyliodid und Propargylalkohol in Gegenwart von Natriumdithionit/Natriumbicarbonat und katalytische Dehalogenierung. Dieses Verfahren ist aus ökonomischen Gründen, aufgrund der hohen Kosten insbesondere für den Platinkatalysator, in technischem Maßstab nicht einsetzbar.

Aus DE-A 41 32 182 und WO 93/13123 ist die Herstellung eines Perfluoralkylalkanols, insbesondere von 4,4,5,5,5-Pentafluor-2-pentan-1-ol, durch Umsetzung von 4,4,5,5,5-Pentafluor-2-penten-1-ol in Gegenwart eines Katalysators mit Wasserstoff bekannt. Die Verfahren weisen die Nachteile auf, dass sie geringe Reaktionsausbeuten liefern.

Alternativ dazu erfolgt die Synthese eines Perfluoralkylalkanols, insbesondere des 4,4,5,5,5-Pentafluor-2-pentan-1-ol, in Acta Chem. Scand. 47, 380 (1993) durch radikalische Umsetzung von Pentaethylfluoriodid mit Allylacetat, Dehalogenierung mit Tributylzinnhydrid und basischer Verseifung. Dieses Verfahren ist im technischen Maßstab nur ineffizient durchzuführen.

Aus Journal Organic Chemistry 26, 2089 (1981) und DE-A 19748775 sind weitere Verfahren zur Herstellung eines Perfluoralkylalkanols ebenfalls durch Umsetzung von Pentafluorethyliodid mit Allylalkohol bekannt. Nachteilig an diesen Verfahren ist, dass sie entweder geringe Reaktionsausbeuten liefern oder ökotoxikologisch bedenklich sind.

Es bestand daher das Bedürfnis nach einem Verfahren, dass die Nachteile des bisherigen Standes der Technik überwindet und mit dem effizient, in technischem Maßstab, in guten Ausbeuten Perfluoralkanole hergestellt werden können.

Überraschend wurde ein Verfahren zur Herstellung von Perfluoralkylalkanolen gefunden, ausgehend von Perfluoralkylbromiden unter Umsetzung mit Allylalkoholen, dass die derzeitigen Nachteile des Standes der Technik überwindet und mit dem Perfluoralkylalkanole in guten Ausbeuten in technischem Maßstab hergestellt werden können.

Gegenstand der Erfindung ist daher ein Verfahren zur Herstellung der Verbindungen der Formel (I) 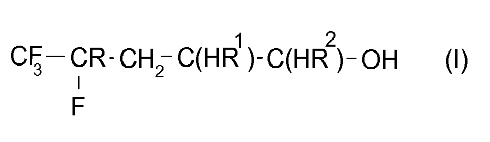 worin R¹ und R² gleich oder verschieden sein können und für Wasserstoff oder C₁-C₁₅-Alkyl steht, das gegebenenfalls weiter durch C₁-C₁₅-Alkoxyreste oder Hydroxylgruppen substituiert sein kann, gegebenenfalls substituiertes C₆-C₁₄-Aryl oder C₆-C₁₄-Arylalkyle steht oder
R¹ und R² zusammen einen 5 - oder 6- gliedrigen carbozyklischen Ring bilden und
R für C₁-C₆-Perfluoralkyl oder Halogen steht
in einem Schritt a) durch Umsetzung von Verbindungen der Formel (II) 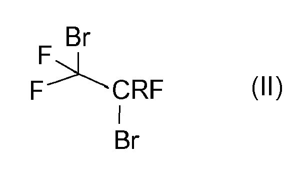 worin R die vorgenannte Bedeutung besitzt,
mit mindestens einem Fluorierungsmittel
in Gegenwart von mindestens einem polaren, aprotischen Lösungsmittel zu Verbindungen der Formel (III) 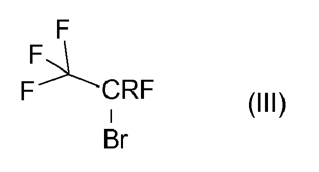 worin R die vorgenannte Bedeutung hat und
in einem Schritt b) die Verbindungen der Formel (III)
in Gegenwart von Radikalinitiatoren mit Verbindungen der Formel (IV), 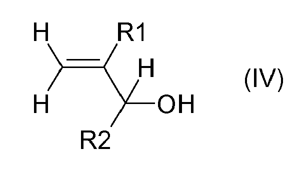 worin R¹ und R² die vorgenannte Bedeutung besitzen, zu Verbindungen der Formel (V) 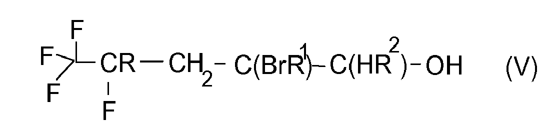 worin R, R¹ und R² die vorgenannte Bedeutung besitzen, umgesetzt werden und in einem Schritt
c) die Verbindungen der Formel (V)
in Gegenwart von mindestens einem Übergangsmetall-Katalysator, mindestens einer Base und mindestens einem Lösungsmittel
mit Wasserstoff
zu Verbindungen der Formel (I) umgesetzt werden.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

R¹ und R² sind unabhängig voneinander und können gleich oder verschieden sein und stehen vorzugsweise für Wasserstoff, (C₁-C₁₀)-Alkyl, das gegebenenfalls weiter durch C₁-C₁₅-Alkoxyreste oder Hydroxylgruppen substituiert sein kann, Phenyl, Biphenyl oder Benzyl, die gegebenenfalls weiter durch Reste substituiert sein können oder R¹ und R² bilden zusammen einen 5- bis 6-gliedrigen carbozyklischen Ring. Bevorzugt stehen R¹ oder/und R² für Wasserstoff, (C₁-C₆)-Alkyl, -(CₙH₂ₙ)-O-(CₐH₂ₐ₊₁), mit n = 1 bis 6 und a = 1 bis 10, für ein einfach oder mehrfach, bevorzugt einfach, durch Hydroxylgruppen, substituiertes (C₁-C₁₀)-Alkyl oder für Phenyl, Biphenyl oder Benzyl die gegebenenfalls weiter durch Reste substituiert sein können. In einer bevorzugten Ausführungsform der Erfindung bilden R¹ und R² zusammen einen 5- bis 6-gliedrigen carbozyklischen Ring. Ganz besonders bevorzugt steht R¹ und R² für Wasserstoff.

Bevorzugt steht R für (C₁-C₆)-Perfluoralkyl, F, Cl, Br. Besonders bevorzugt steht R für Difluormethyl, Trifluormethyl, Pentafluorethyl, Heptafluorisopropyl, Nonafluorbutyl, Chlor oder Fluor. Ganz besonders bevorzugt steht R für Fluor.

Alkyl bzw. Alkoxy steht im Rahmen der Erfindung für einen geradkettigen, zyklischen, verzweigten oder unverzweigten Alkyl- bzw. Alkoxy- Rest mit 1 bis 15 Kohlenstoffatomen. Bevorzugt steht Alkyl-, bzw. Alkoxy-, für einen geradkettigen, zyklischen, verzweigten oder unverzweigten Alkyl-, bzw. Alkoxy-, Rest mit 1 bis 10 Kohlenstoffatomen, ganz besonders bevorzugt mit 1 bis 6 Kohlenstoffatomen.

Beispielhaft und vorzugsweise steht Alkyl für Methyl, Ethyl, n-Propyl, Isopropyl, n-, i-, sek.- oder tert.-Butyl, Cyclopropyl, Cyclobutyl, Cyclopentyl, Cyclohexyl, Cycloheptyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl und n-Decyl. Besonders bevorzugt steht Alkyl für Methyl, Ethyl, n-Propyl, Isopropyl, n-, i-, sek.- oder tert.-Butyl, Cyclopropyl, Cyclobutyl, Cyclopentyl und Cyclohexyl.

Beispielhaft und vorzugsweise steht Alkoxy für Methoxy, Ethoxy, n-Propoxy, Isopropoxy,n-, i-oder tert t-Butoxy, n-Pentoxy, n-Hexoxy, n-Octoxy, cyclo-Hexoxy, n-Pentoxy und n-Decoxy. Besonders bevorzugt steht Alkoxy für Methoxy, Ethoxy, n-Propoxy, Isopropoxy,n-, i- oder t-Butoxy und n-Pentoxy.

Aryl steht im Rahmen der Erfindung für einen mono-, bi- oder trizyklischen carbozyklischen aromatischen Rest mit vorzugsweise 6 bis 14 aromatischen Kohlenstoffatomen (C₆-C₁₄-Aryl). Weiterhin können die carbozyklischen aromatischen Reste mit bis zu fünf gleichen oder verschiedenen Substituenten pro Zyklus substituiert sein, ausgewählt aus der Gruppe Alkyl, Alkanoyloxy, Alkoxy, Alkoxycarbonyl, Monoalkylamino und Dialkylamino. Beispielhaft und bevorzugt steht C₆-C₁₄-Aryl für Biphenyl, Phenyl, Naphthyl, Phenanthrenyl, Anthracenyl oder Fluorenyl.

Alkoxycarbonyl steht im Rahmen der Erfindung vorzugsweise für einen geradkettigen oder verzweigten Alkoxyrest mit 1 bis 6 Kohlenstoffatomen, der über eine Carbonylgruppe verknüpft ist. Beispielhaft und vorzugsweise seien genannt: Methoxycarbonyl, Ethoxycarbonyl, n-Propoxycarbonyl, Isopropoxycarbonyl und t-Butoxycarbonyl.

Alkanoyloxy steht im Rahmen der Erfindung vorzugsweise für einen geradkettigen oder verzweigten Alkylrest mit 1 bis 6 Kohlenstoffatomen, der in der 1-Position ein doppelt gebundenes Sauerstoffatom trägt und in der 1-Position über ein weiteres Sauerstoffatom verknüpft ist. Beispielhaft und vorzugsweise seien genannt: Acetoxy, Propionoxy, n-Butyroxy, i-Butyroxy, Pivaloyloxy und n-Hexanoyloxy.

Arylalkyl bedeutet jeweils unabhängig voneinander einen geradkettigen, zyklischen, verzweigten oder unverzweigten Alkyl-Rest nach vorstehender Definition, der einfach, mehrfach oder vollständig durch Aryl-Reste gemäß vorstehender Definition substituiert sein kann. Ein Beispiel für Arylalkyl ist Benzyl.

Mono- bzw. Dialkylamino bzw. steht im Rahmen der Erfindung für eine Amino-Gruppe die mit einem oder zwei gleichen oder verschiedenen, zyklischen, geradkettigen oder verzweigten Alkylsubstituenten, die vorzugsweise jeweils 1 bis 6 Kohlenstoffatomen aufweisen.

Beispielhaft und vorzugsweise steht Monoalkylamino für Methylamino, Ethylamino, n-Propylamino, Isopropylamino, t-Butylamino, n-Pentylamino und n-Hexylamino.

Beispielhaft und vorzugsweise steht Dialkylamino für *N,N-*Dimethylamino, *N,N-*Diethylamino, *N-*Ethyl-*N-*methylamino, *N-*Methyl-*N-*n-propylamino, *N-*Isopropyl-*N-*n-propylamino, *N-*t-Butyl-*N-*methylamino, *N*-Ethyl-*N*-n-pentylamino und *N*-n-Hexyl-*N*-methylamino.

Perfluoralkyl steht für einen Alkylrest nach vorstehender Definition, der vollständig durch Fluoratome substituiert ist. Beispielhaft und vorzugsweise steht (C₁-C₆)-Perfluoralkyl für 2,2,2-Trifluorethyl, Trifluormethyl, Pentafluorethyl, Heptafluorpropyl, cylco-Nonafluorpentyl, Nonachlorpentyl, Heptafluorisopropyl und Nonafluorbutyl. Bevorzugt steht Perfluoralkyl für Difluormethyl, Trifluormethyl, Pentafluorehtyl, Heptafluorisopropyl und Nonafluorbutyl.

Bevorzugte Verbindung der Formel (I) ist 4,4,5,5,5-Pentafluorpentan-1-ol. Bevorzugte Verbindung der Formel (III) ist Pentafluorethylbromid.

Als Fluoridquellen zur Durchführung des erfindungsgemäßen Verfahrens können beispielsweise HF, Alkalimetall- und Erdalkalimetallfluoride, oder Metallfluoride, wie z.B. NaF, KF, CaF₂, NH₄F oder CuF₂ verwendet werden. Bevorzugt werden als Fluoridquellen HF, Alkalimetall- und Erdalkalimetallfluoride, KHF₂; NH₄F oder Gemische aus diesen Verbindungen verwendet. Besonders bevorzugt wird als Fluoridquelle KF eingesetzt.

Schritt a) des erfindungsgemäßen Verfahrens wird in Gegenwart eines aprotischen, polaren Lösungsmittels, wie beispielsweise und vorzugsweise ausgewählt aus den Gruppen Ether, Ester, Ketone, Nitrile, Amine, Sulfoxide und Sulfolane durchgeführt.

Aprotisch heißt im Rahmen der Erfindung, dass die organischen Lösungsmittel keine Protonen aufweisen, die bezogen auf eine wässrige Vergleichsskala bei 25°C einen pKs-Wert von unter 20 besitzen.
Bevorzugte aprotische, polare Lösungsmittel sind beispielsweise Acetonitril, Benzonitril, Benzylnitril, Dichlormethan, Diethylacetamid, **Diethylether,** Dimethylformamid, Dimethylacetamid, Dimethylketon, Dimethylethylenharnstoff, Dimethylsulfoxid, Diethylketon, N-Methylpyrrolidon, N-Methylcaprolactam, Methyl-tert.-butylketon, Tetramethylharnstoff, Tetraethylharnstoff, Tetramethylensulfon oder Mischungen solcher Lösungsmittel.

Im Rahmen der Erfindung sind Radikalinitiatoren beispielsweise und vorzugsweise ausgewählt aus der Gruppe Peroxide, Dithionite, bevorzugt Natriumdithionit, oder Nitrile oder Gemische dieser Verbindungen. Bevorzugt sind die Radikalinitiatoren Dithionit/Bicarbonat-Gemische, Di-tert.-Butylperoxid, 2,2-Azo-bis-isobuttersäurenitril (AIBN) oder Licht, beispielsweise einer Quecksilber-Hochdruck- oder Wolframfaden-Lampe. Besonders bevorzugt sind die Radikalinitiatoren Dithionit/Bicarbonat-Gemische.

Dithionit/Bicarbonat-Gemische können pro Mol Dithionit z.B. 0,5 bis 2 mol Bicarbonat enthalten. **Vorzugsweise liegt das Verhältnis bei 1:0,7 bis 1,5. Beispielsweise kann soviel** Dithionit/Bicarbonat-Gemisch eingesetzt werden, dass pro Mol Verbindungen der Formel (III) 1 bis 5 mol, vorzugsweise 1,1 bis 3 mol, Dithionit zum Einsatz gelangen.

Organische Radikalinitiatoren können auch in geringeren Mengen eingesetzt werden, beispielsweise 0,01 bis 0,5 mol. Bevorzugt werden 0,05 bis 0,2 mol Radikalinitiator pro Mol Verbindungen der Formel (III) eingesetzt.

Die Reaktion nach Schritt b) des erfindungsgemäßen Verfahrens kann in Gegenwart oder Abwesenheit eines Lösungsmittels durchgeführt werden. Beim Einsatz von Dithionit/Bicarbonat-Gemischen ist es vorteilhaft, Wasser oder Gemische von wasserlöslichen organischen Lösungsmitteln mit Wasser (Wassergehalt vorzugsweise mindestens 30Vol.-%) einzusetzen. Beim Einsatz organischer Radikalinitiatoren oder von Licht können beispielsweise organische, mit Wasser mischbare Lösungsmittel oder auch keine zusätzlichen mit Wasser mischbaren Lösungsmittel eingesetzt werden. Als mit Wasser mischbare Lösungsmittel kommen z.B. in Frage: Nitrile, wie Acetonitril, Proprionitril, n- und i-Butyronitril und Alkohole wie Methanol, Ethanol, n-und i-Propanol, n-, i-, sek.- und tert.-Butanol, Ethandiol, Propan-1,2-diol, Ethoxyethanol, Methoxyethanol, Diethylenglykolmonomethyletherester und Diethylenglykolmonoethylester. Bevorzugt werden als Lösungsmittel Actonitril/Wasser-Gemische eingesetzt. Pro Mol Verbindungen der Formel (III) werden beispielsweise und vorzugsweise 0,5 bis 3 1 Lösungsmittel eingesetzt.

Als Lösungsmittel zur Durchführung von Schritt c) des erfindungsgemäßen Verfahrens kommen z.B. und vorzugsweise Wasser, organische Lösungsmittel und beliebige Mischungen davon in Betracht. Als organische Lösungsmittel seien beispielhaft genannt: aliphatische und alicyclische Kohlenwasserstoffe wie Petrolether, Hexan, Heptan, Cyclohexan und Methylcyclohexan, Ether wie Diethyl-, Diisopropyl-, Methyl-t-butyl- und Methyl-t-amylether, Dioxan, Tetrahydrofuran, 1,2-Dimethoxyethan, 1,2-Diethoxyethan, Diethylenglykoldimethylether und Anisol, Ester wie Methyl-, Ethyl und Butylacetat und Alkohole wie Methanol, Ethanol, n- und i-Propanol, n-, i-, sek.- und tert.-**Butanol, Ethandiol, Propan**-1,2-diol, Ethoxyethanol, Methoxyethanol, Diethylenglykolmonomethylether und Diethylenglykolmonoethylether. Besonders bevorzugt werden als Lösungsmittel in Schritt c) des erfindungsgemäßen Verfahrens Ethylacetat oder Methyl-t-butylether oder Gemische aus diesen Lösungsmittel eingesetzt.

Als Übergangsmetall-Katalysatoren in Schritt c) des erfindungsgemäßen Verfahrens können beispielsweise heterogene Hydrierkatalysatoren wie Platinoxid, Palladium auf Kohle oder Raney-Nickel eingesetzt werden. In einer weiteren bevorzugten Ausführungsform der Erfindung ist das Übergangsmetall des Übergangsmetall-Katalysators Nickel, Rhodium, Ruthenium, Iridium, Platin, Palladium oder deren Salze, besonders bevorzugt sind Nickel, Rhodium, Ruthenium, Platin, Palladium oder deren Salze und Gemische solcher Übergangsmetalle oder deren Salze.

In einer anderen Ausführungsform des erfindungsgemäßen Verfahrens befindet sich das Übergangsmetall bevorzugt auf einem Träger, ausgewählt aus der Gruppe Siliziumdioxid, Aluminiumoxid, Titandioxid, Zeolithe oder Kohlenstoff. Ganz besonders bevorzugt handelt es sich bei dem Träger um Aktivkohle.

In den Fällen, in denen die Übergangsmetall-Katalysatoren anorganisch geträgerte Katalysatoren darstellen, sind Pd-Aktivkohle oder Pt-Aktivkohle bevorzugt.

Bei den eingesetzten geträgerten oder nicht geträgerten Übergangsmetallkatalysatoren handelt es sich um handelsübliche Produkte.

In einer Ausführungsform der Erfindung liegt die Menge des Übergangsmetalls in den Übergangsmetall-Katalysatoren bezogen auf die eingesetzte Masse der geträgerten Übergangsmetall-Katalysatoren, zwischen 0,01 und 99 Gew. %. Bevorzugt liegt die Konzentration zwischen 1 und 60 Gew. %. Besonders bevorzugt zwischen 1 und 10 Gew. %.

In Schritt c) des erfindungsgemäßen Verfahrens kann der Wasserstoff beispielsweise in elementarer Form der Reaktionsmischung zugeführt werden. Der Wasserstoff kann aber auch in situ aus anderen Wasserstoffquellen, wie beispielsweise Hydrazin, Wasserstoffperoxid, Lithiumalanat oder Natriumborhydrid hergestellt werden. Bevorzugt wird der Wasserstoff außerhalb der Reaktionsmischung erzeugt und zugeführt. Beispielsweise und vorzugsweise können 1 bis 10 Moläquivalente Wasserstoff bezogen auf die Menge der Verbindungen der Formel (V) zugegeben werden, bevorzugt werden 1 bis 5 Moläquivalente zugegeben.

Vorzugsweise wird Schritt c) des erfindungsgemäßen Verfahrens in Gegenwart mindestens einer Base durchgeführt. Vorzugsweise wird die Reaktion in Gegenwart mindestens einer Base durchgeführt. Als Basen werden beispielsweise und vorzugsweise Hydrogencarbonate, wie Natrium- und Kaliumhydrogencarbonat und tert. Amine, wie Trimethylamin, Triethylamin, Tributylamin, N,N-Dimethylanilin, N,N-Dimethylbenzylamin, Pyridin, N-Methylpyridin, N-Methylmorpholin, N,N-Dimethylaminopyridin, Diazabicyclooctan (DABCO), Diazabicyclononen (DBN) und Diazabicycloundecen (DBU) oder Gemische dieser Basen eingesetzt. Bevorzugt wird Triethylamin eingesetzt.

Viele der im erfindungsgemäßen Verfahren eingesetzten Verbindungen sind handelsüblich oder können nach analogen, aus dem Stand der Technik bekannten Verfahren hergestellt werden, die dem Fachmann bekannt sind.

In einer Ausführungsform von Schritt a) des erfindungsgemäßen Verfahrens kann dieses beispielsweise auch in Gegenwart oder Abwesenheit von "Ammoniumsalzen", "quartäre Phosphoniumsalzen" und "quartärisierte Guanidine" durchgeführt werden. Beispielsweise und vorzugsweise seien genannt: Tetramethylammoniumchlorid, -bromid, Tetraethylammoniumchlorid, -bromid, Tetrabutylammoniumchlorid, - bromid, Octyl-triethylammoniumchlorid, -bromid, Tetraphenylphosponiumchlorid, -bromid, Hexamethylguanidiniumchlorid, und/oder - bromid oder Gemische solcher Verbindungen.

In einer weiteren Ausführungsform der Erfindung wird Schritt a) bei einer Temperatur im von 100 bis 170°C, bevorzugt von 120 bis 150°C durchgeführt.

Schritt a) der Reaktion wird beispielsweise und vorzugsweise bei einem Druck von 1 bis 200 bar, bevorzugt 1 bis 50 bar durchgeführt.

Beispielsweise und vorzugsweise liegt das Molverhältnis von dem Fluoridquelle zu Verbindungen der Formel (II) zwischen 1 und 100, bevorzugt zwischen 1 und 10, besonders bevorzugt zwischen 1 und 3.

Die Reaktionsdauer in Schritt a) des erfindungsgemäßen Verfahrens beträgt beispielsweise 1 bis 50 Stunden, bevorzugt beträgt die Reaktionsdauer 2 bis 30 Stunden, besonders bevorzugt 2 bis 10 Stunden.

Die Menge an Lösungsmittel ist nur insoweit von Bedeutung, dass die Suspension gut rührbar sein soll, da die gegebenenfalls **zugesetzten Katalysatoren überwiegend die** Fluorierungsgeschwindigkeit beeinflussen.

In einer weiteren Ausführungsform der Erfindung wird Schritt b) bei einer Temperatur von -100°C bis 100°C, bevorzugt von -50°C bis 50°C, besonders bevorzugt von -5°C bis 20°C durchgeführt.

Vorzugsweise wird die Reaktion in Schritt a) im Wesentlichen wasserfrei durchgeführt. Im Wesentlichen wasserfrei bedeutet, dass der Wassergehalt bezogen auf die Menge der eingesetzten Reaktionsmischung vorzugsweise zwischen 0.0001 Gew.% und 0,1 Gew.% liegt.

Schritt b) des erfindungsgemäßen Verfahrens kann beispielsweise unter erhöhtem Druck durchgeführt werden Bevorzugt wird Schritt b) des erfindungsgemäßen Verfahrens bei Drücken von 1 bis 30 bar durchgeführt.

Die molaren Stoffmengenverhältnisse der Verbindungen der Formel (IV) zu den Verbindungen der Formel (III) liegen beispielsweise zwischen 1 : 0,7 bis 20. Bevorzugt beträgt dieses Verhältnis 1 : 0,9 bis 2. Nach Beendigung der Addition wird das Reaktionsgemisch beispielsweise durch Extraktion mit geeigneten Lösungsmitteln, wie beispielsweise Ethylacetat und Methyl-tert.-butylether, aufgearbeitet.

Die Menge an Übergangsmetall in den Übergangsmetall-Katalysatoren bezogen auf die Verbindungen der Formel (V) die in Schritt c) des erfindungemäßen Verfahrens eingesetzt wird, liegt beispielsweise zwischen 0,001 bis 1 Gew.-%, vorzugsweise zwischen 0,01 bis 0,1 Gew.-%.

Die Reaktionsdauer in Schritt b) des erfindungsgemäßen Verfahrens beträgt beispielsweise 1 bis 50 Stunden, bevorzugt beträgt die Reaktionsdauer 2 bis 30 Stunden, besonders bevorzugt 2 bis 10 Stunden.

Schritt c) des erfindungsgemäßen Verfahrens kann beispielsweise und vorzugsweise bei Temperaturen von -20 bis +100°C, bevorzugt bei 0 bis 50°C durchgeführt werden.

Schritt c) des erfindungsgemäßen Verfahrens kann beispielsweise und vorzugsweise bei einem Druck von 1 bis 100 bar, bevorzugt bei einem Druck von 1 bis 50 bar, ganz besonders bevorzugt bei einem Druck von 1 bis 5 bar. Während der Reaktion ist der gewünschte Wasserstoffdruck gegebenenfalls durch Nachdosierung aufrecht zu erhalten.

Die Reaktionsdauer in Schritt c) des erfindungsgemäßen Verfahrens beträgt beispielsweise 1 bis 50 Stunden, bevorzugt beträgt die Reaktionsdauer 2 bis 30 Stunden, besonders bevorzugt 2 bis 10 Stunden.

Die Stoffmengenverhältnisse der Verbindungen der Formel (V) zu den Basen betragen beispielsweise und vorzugsweise 0,9 bis 2 Moläquivalente, vorzugsweise 1 bis 1,5 Moläquivalente.

Bevorzugt wird die Reaktion in einem Autoklaven durchgeführt. Des Weiteren ist bevorzugt unter inerten Bedingungen zu arbeiten. Die Inertisierung kann z.B. durch kontinuierliche oder diskontinuierliche Durchströmung der Reaktionsmischung mit inerten Gasen, wie z.B. Argon und Stickstoff, durchgeführt werden.

Beispielsweise kann in Schritt a) des erfindungsgemäßen Verfahrens zunächst die Lösungsmittel und die Fluorierungsmittel vorgelegt werden und dann die Verbindungen der Formel (II) hinzugegeben werden. Es kann aber z.B. ebenfalls zunächst die Verbindungen der Formel (II) mit den Lösungsmitteln vorgelegt werden und dann die Fluorierungsmittel hinzugegeben werden. Bevorzugt werden zunächst die Lösungsmittel und die Fluorierungsmittel vorgelegt und dann die Verbindungen der Formel (II) hinzugegeben. Beispielsweise und vorzugsweise wir die Reaktion bei erhöhtem Druck durchgeführt. Die Aufarbeitung kann in an sich bekannter Weise, z.B. durch Destillation, erfolgen.

Schritt b) des erfindungsgemäßen Verfahrens kann beispielsweise so durchgeführt werden, dass zunächst die Verbindungen der Formel (IV) und die Verbindungen der Formel (III) in Kontakt gebracht werden und dann die Radikalinitiatoren zugegeben werden. Ebenso können auch z.B. zunächst die Radiakalinitiatoren vorgelegt werden und dann die Verbindungen der Formel (IV) und die Verbindungen der Formel (III) zugegeben werden. Bevorzugt wird Schritt b) des erfindungsgemäßen Verfahrens so durchgeführt, dass zunächst die Verbindungen der Formel (IV) und die Verbindungen der Formel (III) in Kontakt gebracht werden und dann die Radikalinitiatoren zugegeben werden. Die Aufarbeitung kann in an sich bekannter Weise, z.B. durch Extraktion mit bekannten Lösungsmitteln, wie beispielsweise und vorzugsweise Methyl-t-butylether und Ethylacetat, erfolgen.

Beispielsweise und vorzugsweise wird Schritt c) des erfindungsgemäßen Verfahrens so durchgeführt, dass der Übergangsmetall-Katalysator, die Base und das Lösungsmittel, in Gegenwart von Wasserstoff, zunächst vorgelegt werden und dann die Verbindungen der Formel (V) zugegeben werden. Die Zugabe der Verbindungen der Formel (V) erfolgt beispielsweise und vorzugsweise dosiert. Durch die Reaktion kann sich der Wasserstoffdruck ändern. Beispielsweise und vorzugsweise wird die Zugabe von Wasserstoff zur Reaktionsmischung dadurch geregelt, dass der Wasserstoffdruck über den Reaktionszeitraum konstant gehalten wird.

Die Aufarbeitung kann beispielsweise durch Zugabe von Wasser zum Lösen des entstandenen salzförmigen Bromids und anschließende Extraktion des Produktes erfolgen, gegebenenfalls nach Abfiltrieren des Hydrierkatalysators. Gegebenenfalls kann man zur weiteren Reinigung die Rohproduktlösung fraktioniert destillieren.

In einer bevorzugten Ausführungsform der Erfindung werden zunächst die Fluoridquelle (Fluorierungsmittel) in getrocknetem Lösungsmittel mit den Verbindungen der Formel (II) in Kontakt gebracht, Stickstoff aufgedrückt und erwärmt. Nach Beendigung der Reaktionsdauer wird die Verbindung der Formel (III) von der Reaktionsmischung durch Destillation abgetrennt. Verbindungen der Formel (IV) werden zunächst mit dem Lösungsmittel in Kontakt gebracht und die Verbindungen der Formel (III) zugeführt. Danach werden die Radikalinitiatoren hinzugegeben. Nach Ablauf der Reaktion werden die Verbindungen der Formel (V) abgetrennt. Im weiteren Verfahren werden die Übergangsmetall-Katalysatoren, die Lösungsmittel und die Basen vorgelegt und die Verbindungen der Formel (V) hinzugegeben. Danach wird Wasserstoff aufgedrückt und die Reaktionsmischung erwärmt. Die Verbindungen der Formel (I) werden dann durch Destillation abgetrennt und aufgereinigt.

Das erfindungsgemäße Verfahren hat eine Reihe von überraschenden Vorteilen. So benötigt es Hydrierkatalysatoren in üblichen und nicht unverhältnismäßig großen Mengen, es liefert das gewünschte Produkt in hohen Ausbeuten auf technisch einfache Weise, es erfordert nicht die Handhabung von Chemikalien, die wegen ihres Gefahrenpotenzials besonderen Aufwand erfordern, es vermeidet die Anwendung von Ultraschall und es ist ohne Probleme auch in größerem Maßstab durchführbar. Es ist vor allem überraschend, dass das erfindungsgemäße Verfahren die Verbindungen der Formel (I) in hohen Ausbeuten liefert.

Die erfindungsgemäß hergestellten Verbindungen der Formel (I) eignen sich insbesondere als Zwischenprodukte z.B. zur Herstellung von Arzneimitteln.

Daher ist die Verwendung der erfindungsgemäßen Verbindungen der Formel (I) in einem Verfahren zur Herstellung von Arzneimitteln ebenfalls Gegenstand der Erfindung.

Die nachstehenden Beispiele dienen der Erläuterung der Erfindung, ohne dabei auf dieses beschränkt zu sein.

### Beispiele

### 1. Herstellung von Pentafluorethylbromid

1400 ml Tetramethylensulfon werden zusammen mit 189 g Kaliumfluorid (3,23 mol) in einem Autoklaven vorgelegt und der Ansatz durch Abdestillieren von 150 ml Lösungsmittel getrocknet. Anschließend werden 554 g (2.13 mol) 1,2-Dibrom-tetrafluorethan zugetropft und der Ansatz unter 4 bar Stickstoff gesetzt und auf 130°C aufgeheizt, wobei sich ein Druck von 19 bar einstellt. Der Ansatz wird bei dieser Temperatur noch für zwei Stunden gerührt und dann die Temperatur in drei Stunden auf 175°C gesteigert. Danach wird auf 0°C abgekühlt, entspannt und das Produkt aus dem Reaktionsgemisch in eine auf -35°C gekühlte Vorlage (Druckbehältnis) überkondensiert. Es werden 302 g (1,52 mol, 71% d.Th.) Pentafluorethylbromid erhalten.

### 2. Herstellung von 4,4,5,5,5-Pentafluor-2-brom-1-pentanol

Es werden 1000 ml Wasser zusammen mit 1200 ml Acetonitril, 74.2 g (1.28 mol) Allylalkohol unter Rühren in einem Druckbehälter vorgelegt und auf -15°C abgekühlt. Anschließend werden unter Rühren 252 g (1,25 mol) Pentafluorethylbromid aus einer auf einer Waage stehenden Druckgasflasche eingeleitet. Hierzu wurde eine Mischung aus 248 g (1,35 mol) 95gew.-%igen Natriumdithionit und 121 g (1,44 mol) Natriumhydrogencarbonat auf einmal zugegeben und die Reaktionsmischung unter Rühren für ca. 2 h auf einer Temperatur von unter 0°C gehalten. Man lässt die Reaktionsmischung langsam auf 20°C erwärmen, entspannt den Druckbehälter und gibt die erhaltene Suspenion auf 1500 ml Wasser. Anschließend wird zweimal mit je 600 ml Ethylacetat extrahiert, die vereinten organischen Phasen werden getrocknet und eingedampft. Nach vollständigem Abziehen des Lösungsmittels werden 273 g (1.05 mol; 84% d. Th.; GC: > 98,7%) 4,4,5,5,5-Pentafluor-2-brom-1-pentanol als bräunliches Öl erhalten.

### 3. Herstellung von 4,4,5,5,5-Pentafluorpentan-1-ol

In einem Rührautoklaven werden 500ml Ethylacetat, 135 ml (0.974 mol) Triethylamin und 2.95 g Palladium auf Kohle (5 Gew.-% Palladium) vorgelegt und 52 bar Wasserstoff aufgedrückt. Über mehrere Stunden werden 212 g (0.825 mol) 4,4,5,5,5-Pentafluor-2-brom-1-pentanol zudosiert und bei einer Reaktionstemperatur von 35°C der Wasserstoffdruck auf 40 bis 60 bar gehalten. Nach vollständiger Zugabe wurde noch 2 h unter Rühren nachhydriert, bis der Umsatz gemäß GC-Kontrolle vollständig war. Nach Abkühlen des Autoklaven auf 20°C wird dieser vorsichtig entspannt. Zur Reaktionsmischung werden 400 ml Wasser gegeben und der Katalysator durch Filtration abgetrennt. Das Filtrat wird mehrfach mit Ethylacetat extrahiert und die vereinten organischen Phasen getrocknet. Das Lösungsmittel wird bis zu einem Vakuum von 150 mbar weitestgehend abgezogen, die anschließende Reinigung mittels fraktionierter Destillation (Sdp. 132.5°C) liefert 195 g (0,759 mol, 91% d.Th.) 4,4,5,5,5-Pentafluorpentan-1-ol mit einer Reinheit von 97% laut GC-Analyse.

## Patentansprüche

1. Verfahren zur Herstellung von Verbindungen der Formel (I), 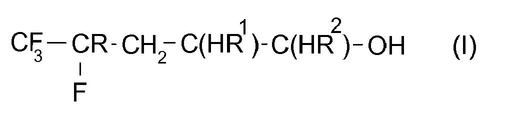 worin R¹ und R² gleich oder verschieden sein können und für Wasserstoff oder C₁-C₁₅-Alkyl steht, dass gegebenenfalls weiter durch C₁-C₁₅-Alkoxyreste oder Hydroxylgruppen substituiert sein kann, oder gegebenenfalls substituiertes, C₆-C₁₄-Aryl oder C₆-C₁₄-Arylalkyl, darstellen oder
R¹ und R² zusammen einen 5 - oder 6- gliedrigen carbozyklischen Ring bilden und
R für C₁-C₆-Perfluoralkyl oder Halogen steht,
**dadurch gekennzeichnet, dass**
in einem Schritt a) Verbindungen der Formel (II) 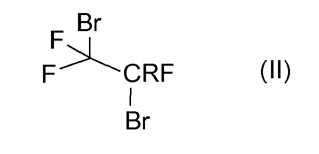 worin R die vorgenannte Bedeutung besitzt,
mit mindestens einer Fluoridquelle
in Gegenwart von mindestens einem polaren, aprotischen Lösungsmittel zu Verbindungen der Formel (III) 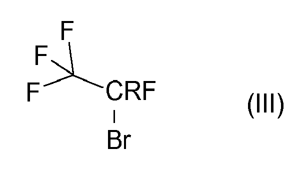 worin R die vorgenannte Bedeutung hat, umgesetzt werden und
in einem Schritt b) die Verbindungen der Formel (III)
in Gegenwart von Radikalinitiatoren mit Verbindungen der Formel (IV), 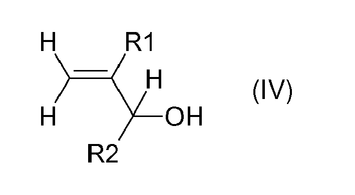 worin R¹ und R² die vorgenannte Bedeutung besitzen, zu Verbindungen der Formel (V) 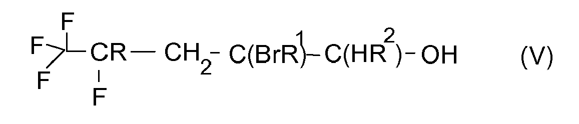 worin R, R¹ und R² die vorgenannte Bedeutung besitzen, umgesetzt werden und in einem Schritt
c) die Verbindungen der Formel (V)
in Gegenwart von mindestens einem Übergangsmetall-Katalysator, mindestens einer Base
und mindestens eines Lösungsmittels
mit Wasserstoff
zu Verbindungen der Formel (I) umgesetzt werden.

2. Verfahren nach Anspruch 1, **dadurch gekennzeichnet, dass** R für (C₁-C₆)-Perfluoralkyl oder F, Cl oder Br steht.

3. Verfahren nach einem der Ansprüche 1 oder 2, **dadurch gekennzeichnet, dass** R¹ und R² gleich und verschieden sind und unabhängig voneinander für Wasserstoff, (C₁-C₆)-Alkyl,
-(CₙH₂ₙ)-O-(CₐH₂ₐ₊₁), mit n = 1 bis 6 und a = 1 bis 10, oder für ein einfach oder mehrfach durch Hydroxylgruppen substituiertes (C₁-C₁₀)-Alkyl und/oder für Phenyl, Biphenyl oder Benzyl stehen.

4. Verfahren nach einem oder mehreren der Ansprüche 1 bis 3 **dadurch gekennzeichnet, dass** die Fluoridquelle NaF, KF, CaF₂ und/oder NH₄F, KHF_{2;}

5. Verfahren nach einem oder mehreren der Ansprüche 1 bis 4 **dadurch gekennzeichnet, dass** das organische, aprotische polare Lösungsmittel aus Schritt a) Acetonitril, Benzonitril, Benzylnitril, Dichlormethan, Diethylacetamid, Diethylether, Dimethylformamid, Dimethylacetamid, Dimethylketon, Dimethylethylenharnstoff, Dimethylsulfoxid, Diethylketon, N-Methylpyrrolidon, N-Methylcaprolactam, Methyl-tert.-butylketon, Tetramethylharnstoff, Tetraethylharnstoff, oder Tetramethylensulfon ist.

6. Verfahren nach einem oder mehreren der Ansprüche 1 bis 5, **dadurch gekennzeichnet, dass** die Radikalinitiatoren Dithionit/Bicarbonat-Gemische sind.

7. Verfahren nach einem oder mehreren der Ansprüche 1 bis 6, **dadurch gekennzeichnet, dass** als Übergangsmetall-Katalysatoren Platinoxid, Palladium auf Kohle oder Raney-Nickel eingesetzt werden.

8. Verfahren nach einem oder mehreren der Ansprüche 1 bis 7, **dadurch gekennzeichnet, dass** die **Reaktion in Schritt a) in Gegenwart** von Tetramethylammoniumchlorid, -bromid, Tetraethylammoniumchlorid, -bromid, Tetrabutylammoniumchlorid, - bromid, Octyl-triethylammoniumchlorid, -bromid, Tetraphenylphosponiumchlorid, -bromid, Hexamethylguanidiniumchlorid, und/oder - bromid durchgeführt wird.

9. Verfahren nach einem oder mehreren der Ansprüche 1 bis 9, **dadurch gekennzeichnet, dass** als Base Natrium- und Kaliumhydrogencarbonat, Trimethylamin, Triethylamin, Tributylamin, N,N-Dimethylanilin, N,N-Dimethylbenzylamin, Pyridin, N-Methylpyridin, N-Methylmorpholin, N,N-Dimethylaminopyridin, Diazabicyclooctan (DABCO), Diazabicyclononen (DBN) und/oder Diazabicycloundecen (DBU) eingesetzt werden.

10. Verfahren nach einem oder mehreren der Ansprüche 1 bis 10, **dadurch gekennzeichnet, dass** Schritt b) bei einer Temperatur von -5°C bis 20°C durchgeführt wird.

11. Verfahren nach einem oder mehreren der Ansprüche 1 bis 11, **dadurch gekennzeichnet dass** Schritt a) bei Temperaturen von 120°C bis 150°C durchgeführt wird.

12. Verfahren nach einem oder mehreren der Ansprüche 1 bis 12, **dadurch gekennzeichnet, dass** bei dem Radikalinitiator das Stoffmengenverhältnis zwischen Dithionit und Bicarbonat in dem Radikalinitiator zwischen 1:0,7 bis 1,5 beträgt.

13. Verfahren nach einem oder mehreren der Ansprüche 1 bis 13, **dadurch gekennzeichnet, dass** der Reaktionsdruck in Schritt c) zwischen 1 bis 50 bar liegt.
